# EUROPEAN PATENT APPLICATION

(11) **EP 4 682 249 A1**
(43) Date of publication of application: **21.01.2026**
(21) Application number: 24770953.8
(22) Date of filing: 14.03.2024
(51) Int. Cl.: C12N 5/0793, C12N 1/00, C12N 5/10, C12N 5/079, C12Q 1/02, C12Q 1/06

(54) **METHOD FOR PRODUCING NERVE CELLS DAMAGED BY OXIDATION STRESS AND APPLICATION THEREOF**

(30) Priority: 14.03.2023 JP 2023039576; 31.01.2024 JP 2024012581
(71) Applicant: FUJIFILM Corporation, Tokyo 106-8620 (JP)
(72) Inventor: ENDOH Setsu, Ashigarakami-gun, Kanagawa 258-8577 (JP); KOBAYASHI Hayato, Ashigarakami-gun, Kanagawa 258-8577 (JP); SHIN Euikyung, Ashigarakami-gun, Kanagawa 258-8577 (JP); TANABE Hirokazu, Ashigarakami-gun, Kanagawa 258-8577 (JP); KATO Hiroshi, Ashigarakami-gun, Kanagawa 258-8577 (JP); MASUYAMA Hitoshi, Ashigarakami-gun, Kanagawa 258-8577 (JP)
(74) Representative: Hoffmann Eitle
(86) International application number: PCT/JP2024/009911
(87) International publication number: WO 2024/190850

(57) **Abstract**

An object of the present invention is to provide a method for producing nerve cells damaged by oxidative stress from a human-derived pluripotent stem cell; a culturing method for cells that allows nerve cells damaged by oxidative stress to be produced from a human-derived pluripotent stem cell; nerve cells damaged by oxidative stress; an evaluation method for a test substance, a screening method for a drug for prevention and/or treatment of a neurodegenerative disease, a screening method for a necroptosis inhibitor, and a screening method for a ferroptosis inhibitor, which use the nerve cells.

According to the present invention, there is provided a production method of nerve cells damaged by oxidative stress, the method including a step a of seeding nerve cells obtained by differentiation from a human-derived pluripotent stem cell, at a cell density of 20.0 × 10⁴ cells/cm² or less by using a culture medium that substantially does not contain an antioxidant and substantially does not contain an oxidant and a step b of culturing the nerve cells by using the culture medium that substantially does not contain an antioxidant and substantially does not contain an oxidant.

## Description

### Technical Field

The present invention relates to a production method of nerve cells damaged by oxidative stress and a culturing method for the cells. The present invention further relates to nerve cells damaged by oxidative stress. The present invention further relates to an evaluation method for a test substance, a screening method for a drug for prevention and/or treatment of a neurodegenerative disease, a screening method for a necroptosis inhibitor, and a screening method for a ferroptosis inhibitor, all using the above-described nerve cells.

### Background Art

Neurodegenerative diseases such as Alzheimer's disease (AD) and amyotrophic lateral sclerosis (ALS) are diseases in which nerve cells are gradually damaged and lost (Patent Documents 1 and 2), and it is known that oxidative stress is significantly involved in the pathogenic mechanism thereof (Non-Patent Documents 3 and 4). In vitro studies on human neurodegenerative diseases have conventionally used rat or mouse cells because obtaining human central nervous system cells has been difficult. However, in recent years, since the invention of induced pluripotent stem cells (iPS cells), it has become relatively easy to obtain human central nervous system cells, including nerve cells, and this has greatly contributed to research on human neurodegenerative diseases (Non-Patent Documents 5 to 7).

The effect of oxidative stress on iPS cell-derived nerve cells related to neurodegenerative diseases has been studied. For example, in the study by Feng-Lan Chiu et al., oxidative stress is applied to iPS cell-derived nerve cells by treating with hydrogen peroxide for 6 hours, and it reproduces neuronal damage of neurodegenerative diseases (Non-Patent Document 8). In addition, Patent Document 1 describes that treatment with arsenite for 16 hours induces damage in motor neurons produced from iPS cells derived from familial ALS patients. Patent Document 2 describes that motor neurons produced from human-derived iPS cells undergo cellular damage on transient treatment with hydrogen peroxide. However, since nerve damage is induced by short-time oxidative stress stimulus under conditions in which an oxidizing agent such as hydrogen peroxide is used, whereas neurodegenerative disease is a disease in which nerve cells are gradually lost over a long period of time, there is a possibility that the pathological condition of the neurodegenerative disease as it occurs in vivo may not be accurately reproduced.

Therefore, there is a study in which chronic application of weak oxidative stress reproduces neuronal damage of neurodegenerative diseases (Non-Patent Document 9). In this study, it is considered that familial neurodegenerative disease is reproduced since nerve damage is induced only in a case where a weak oxidative stress is applied and a disease-related gene is knocked out. In addition, in Patent Document 3 and Non-Patent Document 10, it is described that oxidative stress is enhanced and neuronal damage is induced by culturing the cerebral cortical neurons produced from iPS cells derived from familial AD patients in a culture medium that does not contain an antioxidant.

However, it is known that in neurodegenerative diseases, for example, 95% of AD is sporadic (Non-Patent Document 11) and 90% of ALS is sporadic (Non-Patent Document 12), and most patients have no mutation in disease-related genes. Therefore, an evaluation system capable of easily reproducing the pathological condition of sporadic neurodegenerative disease is required, but sufficient results have not been obtained.

### Prior Art Documents

### Patent Documents

Patent Document 1: JP2015-506905A
Patent Document 2: JP2019-037245A
Patent Document 3: WO2013/140327A

### Non-Patent Documents

Non-Patent Document 1: Brittany N Dugger et al., Cold Spring Harbor Perspectives in Biology, vol. 9, Article No. a028035, 2017
Non-Patent Document 2: Hao Chi et al., International Journal of Molecular Sciences, vol. 19, Article No. 3082, 2018
Non-Patent Document 3: Giovanna Cenini et al., Oxidative Medicine and Cellular Longevity, vol. 2019, Article No. 2105607, 2019
Non-Patent Document 4: Vanessa Castelli et al., Frontiers in Molecular Neuroscience, vol. 12, Article No. 132, 2019
Non-Patent Document 5: Okano et al., Molecular Brain, vol. 7, No. 22, pp. 1 to 12, 2014
Non-Patent Document 6: Valadez-Barba et al., Regenerative Therapy, vol. 15, pp. 332 to 339, 2020
Non-Patent Document 7: Zeng et al., STEM CELLS TRANSLATIONAL MEDICINE, vol. 3, pp. 1418 to 1428, 2014
Non-Patent Document 8: Feng-Lan Chiu et al., Human Molecular Genetics, vol. 24, pp. 6066 to 6079, 2015
Non-Patent Document 9: Ruilin Tian et al., Nature Neuroscience, vol. 24, pp. 1020 to 1034, 2021
Non-Patent Document 10: Kondo et al., Cell Stem Cell, vol. 12, pp. 487 to 496, 2013
Non-Patent Document 11: Jitin Bali et al., Proceedings of the National Academy of Sciences of the United States of America, vol. 109, pp. 15307 to 15311, 2012
Non-Patent Document 12: Francois Gros-Louis et al., Biochemica et Biophysica Acta, vol. 1762, pp. 956 to 972, 2006

### Summary of Invention

### Object to be solved by the invention

An object to be achieved of the present invention is to provide a method for producing nerve cells damaged by oxidative stress from a human-derived pluripotent stem cell, and a culturing method for cells that allows nerve cells damaged by oxidative stress to be produced from a human-derived pluripotent stem cell. Another object to be achieved of the present invention is to provide nerve cells damaged by oxidative stress. Another object to be achieved of the present invention is to provide an evaluation method for a test substance, a screening method for a drug for prevention and/or treatment of a neurodegenerative disease, a screening method for a necroptosis inhibitor, and a screening method for a ferroptosis inhibitor, all using the above-described nerve cells.

### Means for solving the object

As a result of intensive studies to achieve the above objects, the present inventors have found that nerve cells damaged by oxidative stress can be produced by seeding nerve cells obtained by differentiation from a human-derived pluripotent stem cell, at a cell density of 20.0 × 10⁴ cells/cm² or less by using a culture medium that substantially does not contain an antioxidant and substantially does not contain an oxidant and further culturing the nerve cells by using the culture medium that substantially does not contain an antioxidant and substantially does not contain an oxidant. The present invention has been completed based on these findings.

That is, according to an aspect of the present invention, the following invention is provided.
<1> A production method of nerve cells damaged by oxidative stress, the production method comprising a step a of seeding nerve cells obtained by differentiation from a human-derived pluripotent stem cell, at a cell density of 20.0 × 10⁴ cells/cm² or less by using a culture medium that substantially does not contain an antioxidant and substantially does not contain an oxidant and a step b of culturing the nerve cells by using the culture medium that substantially does not contain an antioxidant and substantially does not contain an oxidant.
<2> The production method according to <1>, in which the cell density is 0.2 × 10⁴ cells/cm² or more and 20.0 × 10⁴ cells/cm² or less.
<3> The production method according to <1> or <2>, in which the human-derived pluripotent stem cell is a pluripotent stem cell having no mutation in a disease-related gene.
<4> The production method according to any one of <1> to <3>, in which the nerve cells are motor neurons, cortical excitatory neurons, or substantia nigra neurons.
<5> The production method according to any one of <1> to <4>, in which the nerve cells damaged by oxidative stress satisfy at least one or more of the following (i) to (iv):
   (i) a marker related to the oxidative stress is positive,
   (ii) neurites are retracted,
   (iii) necroptosis is induced, and
   (iv) ferroptosis is induced.
<6> A culturing method for cells, comprising a step a of seeding nerve cells obtained by differentiation from a human-derived pluripotent stem cell, at a cell density of 20.0 × 10⁴ cells/cm² or less by using a culture medium that substantially does not contain an antioxidant and substantially does not contain an oxidant and a step b of culturing the nerve cells by using the culture medium that substantially does not contain an antioxidant and substantially does not contain an oxidant.
<7> The culturing method according to <6>, in which the cell density is 0.2 × 10⁴ cells/cm² or more and 20.0 × 10⁴ cells/cm² or less.
<8> The culturing method according to <6> or <7>, in which the human-derived pluripotent stem cell is a pluripotent stem cell having no mutation in a disease-related gene.
<9> The culturing method according to any one of <6> to <8>, in which the nerve cells are motor neurons, cortical excitatory neurons, or substantia nigra neurons.
<10> Nerve cells obtained by the production method according to <1>, which satisfy at least one of the following (i) to (iv):
   (i) a marker related to the oxidative stress is positive,
   (ii) neurite length is retracted,
   (iii) necroptosis is induced, and
   (iv) ferroptosis is induced.
<11> The cells according to <10>, in which the nerve cells are motor neurons, cortical excitatory neurons, or substantia nigra neurons.
<12> An evaluation method for a test substance, comprising bringing the test substance into contact with the nerve cells according to <10>.
<13> The evaluation method for a test substance according to <12>, further comprising producing the nerve cells according to <10> by the method according to <1>, in which the test substance is brought into contact with the nerve cells.
<14> A screening method for a drug for prevention and/or treatment of a neurodegenerative disease, the method comprising bringing a test substance into contact with the nerve cells according to <10>.
<15> The screening method according to <14>, further comprising producing the nerve cells according to <10> by the method according to <1>, in which the test substance is brought into contact with the nerve cells.
<16> The screening method according to <14>, in which the neurodegenerative disease is selected from the group consisting of Alzheimer's disease (AD), spinocerebellar degeneration, frontotemporal lobar degeneration (FTLD), Parkinson's disease, amyotrophic lateral sclerosis (ALS), dementia with Lewy bodies, Huntington's disease, and Niemann-Pick disease.
<17> The screening method according to <14>, further comprising, after bringing the test substance into contact with the nerve cells according to<10>, (A) a step of culturing the nerve cells that has been brought into contact with the test substance and nerve cells, used as a control, that have never been brought into contact with the test substance, (B) a step of measuring nerve damage in the nerve cells, and (C) a step of selecting, as a candidate for a drug for prevention and/or treatment of a neurodegenerative disease, a test substance that suppresses the nerve damage in comparison with the nerve cells, used as a control, that have never been brought into contact with the test substance.
<18> The screening method according to <17>, in which the step (B) is a step of measuring the number and/or the neurite length of the nerve cells obtained in the step (A).
<19> The method according to <17> or <18>, in which the step (C) is a step of selecting, as a candidate for a drug for prevention and/or treatment of a neurodegenerative disease, a test substance that has been brought into contact with the nerve cells and causes the number and/or the neurite length of the nerve cells to be greater than that in the control.
<20> A screening method for a necroptosis inhibitor, comprising (1) a step of bringing a test substance into contact with the nerve cells according to <10>, (2) a step of culturing the nerve cells that has been brought into contact with the test substance in the step (1) and nerve cells, used as a control, that have never been brought into contact with the test substance, (3) a step of measuring nerve damage in the nerve cells, and (4) a step of selecting, as a candidate for a necroptosis inhibitor, a test substance that suppresses the nerve damage in comparison with the nerve cells, used as a control, that have never been brought into contact with the test substance.
<21> The screening method according to <20>, further comprising producing the nerve cells according to <10> by the method according to <1>, in which the test substance is brought into contact with the nerve cells.
<22> A screening method for a ferroptosis inhibitor, comprising (1) a step of bringing a test substance into contact with the nerve cells according to <10>, (2) a step of culturing the nerve cells that has been brought into contact with the test substance in the step (1) and nerve cells, used as a control, that have never been brought into contact with the test substance, (3) a step of measuring nerve damage in the nerve cells, and (4) a step of selecting, as a candidate for a ferroptosis inhibitor, a test substance that suppresses the nerve damage in comparison with the nerve cells, used as a control, that have never been brought into contact with the test substance.
<23> The screening method according to <22>, further comprising producing the nerve cells according to <10> by the method according to <1>, in which the test substance is brought into contact with the nerve cells.

### Effect of the invention

According to the present invention, it is possible to produce nerve cells damaged by oxidative stress from a human-derived pluripotent stem cell.

### BRIEF DESCRIPTION OF THE DRAWINGS

[Fig. 1] Fig. 1 shows the results of quantifying the neurite length over time in nerve cells after the culture medium was exchanged at 48 hours of culture.
[Fig. 2] Fig. 2 shows the results of quantifying the neurite length over time in nerve cells after the culture medium was exchanged at 72 hours of culture.
[Fig. 3] Fig. 3 shows the results of quantifying the neurite length over time in nerve cells after the culture medium was exchanged at 96 hours of culturing.
[Fig. 4] Fig. 4 shows the results of quantifying the neurite length over time in nerve cells when seeded at a cell seeding density of 0.3 × 10⁴ cells/cm².
[Fig. 5] Fig. 5 shows the results of quantifying the neurite length over time in nerve cells when seeded at a cell seeding density of 0.9 × 10⁴ cells/cm².
[Fig. 6] Fig. 6 shows the results of quantifying the neurite length over time in nerve cells when seeded at a cell seeding density of 1.9 × 10⁴ cells/cm².
[Fig. 7] Fig. 7 shows the results of quantifying the neurite length over time in nerve cells when seeded at a cell seeding density of 4.7 × 10⁴ cells/cm².
[Fig. 8] Fig. 8 shows the results of quantifying the neurite length over time in nerve cells when seeded at a cell seeding density of 9.4 × 10⁴ cells/cm².
[Fig. 9] Fig. 9 shows the results of quantifying the neurite length over time in nerve cells when seeded at a cell seeding density of 12.5 × 10⁴ cells/cm².
[Fig. 10] Fig. 10 shows the results of quantifying the neurite length over time in nerve cells cultured in BrainPhys medium used as the basal medium.
[Fig. 11] Fig. 11 shows the results of quantifying the neurite length over time in nerve cells cultured in Neurobasal medium used as the basal medium.
[Fig. 12] Fig. 12 shows the results of quantifying the amount of LDH in the culture medium from the 3rd day to the 6th day of culture.
[Fig. 13] Fig. 13 shows the results of quantifying the intracellular ROS of the nerve cells after 6 days of culture.
[Fig. 14] Fig. 14 shows the results of quantifying the neurite length over time in nerve cells treated with a necroptosis inhibitor (Necrostatin-1) and images of representative cells. (A) Change in neurite length over time, (B to D) images on the 6th day of culture, (B) Stress(-), (C) Stress(+), and (D) 20 µM Necrostatin-1
[Fig. 15] Fig. 15 shows the results of quantifying the neurite length over time in nerve cells treated with a ferroptosis inhibitor (Ferrostatin-1).
[Fig. 16] Fig. 16 shows the results of quantifying the neurite length over time in nerve cells treated with a ferroptosis inhibitor (Liproxstatin-1).
[Fig. 17] Fig. 17 shows the results of quantifying the neurite length over time in nerve cells treated with a ferroptosis inhibitor (UAMC-3203).
[Fig. 18] Fig. 18 shows the results of calculating the area under the curve (AUC) from the curve of the change in neurite length over time for 14 days in nerve cells treated with various compounds from the compound library.
[Fig. 19] Fig. 19 shows the results of quantifying the neurite length over time in nerve cells treated with an ALS therapeutic drug (Edaravone).

### Embodiments for carrying out the invention

Hereinafter, embodiments of the present invention will be specifically described.

The present invention relates to a production method of nerve cells damaged by oxidative stress, the method comprising a step a of seeding nerve cells obtained by differentiation from a human-derived pluripotent stem cell, at a cell density of 20.0 × 10⁴ cells/cm² or less by using a culture medium that substantially does not contain an antioxidant and substantially does not contain an oxidant and a step b of culturing the nerve cells by using the culture medium that substantially does not contain an antioxidant and substantially does not contain an oxidant. That is, in the present invention, by chronically applying weak oxidative stress to the nerve cells produced from the human-derived pluripotent stem cell, it is possible to produce nerve cells damaged by oxidative stress.

The present invention further relates a culturing method for cells, comprising a step a of seeding nerve cells, which have been induced from a human-derived pluripotent stem cell, at a cell density of 20.0 × 10⁴ cells/cm² or less by using a culture medium that substantially does not contain an antioxidant and substantially does not contain an oxidant and a step b of culturing the nerve cells by using the culture medium that substantially does not contain an antioxidant and substantially does not contain an oxidant. That is, in the present invention, by chronically applying weak oxidative stress to the nerve cells, which have been induced to differentiate from the human-derived pluripotent stem cell, it is possible to culture nerve cells damaged by oxidative stress.

In the production method of nerve cells and the culturing method for cells according to the embodiment of the present invention, the cell density at the time of seeding is only required to be 20.0 × 10⁴ cells/cm² or less. In the related art, to induce a nerve damage due to oxidative stress, it is necessary to add an exogenous oxidant or to use a nerve cell derived from a patient with a mutation in a disease-related gene. In the present invention, a cell density that enables induction of cellular damage due to chronic oxidative stress is found even in a case of nerve cells produced from an iPS cell derived from a sample having no mutation in a disease-related gene.

From the viewpoint of using the cells obtained by the production method and the culturing method for cells according to the embodiment of the present invention for the evaluation of the test substance, it is preferable that the cell density at the time of seeding is set to a range in which the presence or absence of cellular damage due to chronic oxidative stress can be determined. The lower limit value of the cell density is not particularly limited, but is, for example, preferably 0.2 × 10⁴ cells/cm² or more, more preferably 0.3 × 10⁴ cells/cm² or more, still more preferably 1.0 × 10⁴ cells/cm² or more, even still more preferably 2.0 × 10⁴ cells/cm² or more, particularly preferably 3.0 × 10⁴ cells/cm² or more, and most preferably 4.0 × 10⁴ cells/cm² or more. The upper limit value of the cell density may be, for example, 20.0 × 10⁴ cells/cm² or less, and is preferably less than 20.0 × 10⁴ cells/cm², more preferably 12.5 × 10⁴ cells/cm² or less, still more preferably 12.0 × 10⁴ cells/cm² or less, even still more preferably 11.0 × 10⁴ cells/cm² or less, and particularly preferably 10.0 × 10⁴ cells/cm² or less. The cell density is preferably 0.2 × 10⁴ cells/cm² or more and 20.0 × 10⁴ cells/cm² or less, more preferably 0.3 × 10⁴ cells/cm² or more and 20.0 × 10⁴ cells/cm² or less, still more preferably 1.0 × 10⁴ cells/cm² or more and 20.0 × 10⁴ cells/cm² or less, even still more preferably 3.0 × 10⁴ cells/cm² or more and 12.5 × 10⁴ cells/cm² or less, and particularly preferably 4.0 × 10⁴ cells/cm² or more and 10.0 × 10⁴ cells/cm² or less.

Examples of the human-derived pluripotent stem cell include human induced pluripotent stem cells (human iPS cells), human embryonic stem cells (human ES cells), human mesenchymal stem cells, and the like. Human iPS cells are preferable, but the human-derived pluripotent stem cell is not particularly limited thereto. The human iPS cell is an iPS cell produced from a human cell.

The human-derived pluripotent stem cell is preferably a pluripotent stem cell having no mutation in a disease-related gene. The "no mutation in disease-related gene" means that there is no mutation that causes a nervous system disease in the disease-related gene. That is, in a case where there is a mutation in a gene but the mutation does not cause a disease, it is interpreted that there is no mutation in the disease-related gene.

The ES cell can be established, for example, by culturing an early embryo before implantation, an inner cell mass constituting the early embryo, a single blastomere, or the like (Manipulating the Mouse Embryo A Laboratory Manual, Second Edition, Cold Spring Harbor Laboratory Press, 1994, Thomson, J. A. et al., Science, 282, 1145-1147, 1998). As the early embryo, an early embryo produced by nuclear transplantation of a nucleus of a somatic cell may be used (Wilmut et al., Nature, 385, 810, 1997, Cibelli et al., Science, 280, 1256, 1998, Iriya et al., Protein Nucleic Acid Enzyme, 44, 892, 1999, Baguisi et al., Nature Biotechnology, 17, 456, 1999, Wakayama et al., Nature, 394, 369, 1998, Nature Genetics, 22, 127, 1999, and Proc. Natl. Acad. Sci. USA, 96, 14984, 1999, Rideout III et al., Nature Genetics, 24, 109, 2000, Tachibana et al., Human Embryonic Stem Cells Derived by Somatic Cell Nuclear Transfer, Cell, 2013, in press). As the early embryo, a parthenogenetic embryo may be used (Kim et al., Science, 315, 482-486, 2007, Nakajima et al., Stem Cells, 25, 983-985, 2007, Kim et al., Cell Stem Cell, 1, 346-352, 2007, Revazova et al., Cloning Stem Cells, 9, 432-449, 2007, Revazova et al., Cloning Stem Cells, 10, 11-24, 2008). In addition to the above-described papers, the production of the ES cell is described in Strelchenko N. et al., Reprod Biomed Online. 9, 623-629, 2004, Klimanskaya I. et al., Nature 444, 481-485, 2006, Chung Y. et al., Cell Stem Cell 2, 113-117, 2008, Zhang X. et al., Stem Cells 24, 2669-2676, 2006, Wassarman, P. M. et al., Methods in Enzymology, Vol. 365, 2003, or the like.

It is noted that a fused ES cell obtained by cell fusion of an ES cell with a somatic cell is also included in the embryonic stem cell that is used in the method according to the embodiment of the present invention.

Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESIBIO, and the like.

The iPS cell means a cell that is produced by reprogramming a somatic cell by introducing reprogramming factors and has pluripotency (multiple differentiation potency) and proliferation ability. The iPS cells exhibit properties similar to the ES cells. The somatic cells used for producing iPS cells are not particularly limited and may be differentiated somatic cells or undifferentiated stem cells. The iPS cell can be prepared by a known method or the like. In addition, it is naturally expected that an iPS cell production method to be developed in the future will be applied.

The most basic producing method for an iPS cell is a method in which four transcription factors, Oct3/4, Sox2, Klf4, and c-Myc are introduced into a cell using a virus (Takahashi K., Yamanaka S., Cell 126 (4), 663-676, 2006, Takahashi, K., et al., Cell 131 (5), 861-72, 2007). It has been reported that human iPS cells have been established by introducing four factors, Oct4, Sox2, Lin28, and Nanog (Yu J, et al.: Science 318 (5858), 1917-1920, 2007). It has also been reported that iPS cells have been established by introducing three factors excluding c-Myc (Nakagawa M, et al.: Nat. Biotechnol. 26 (1), 101-106, 2008), two factors of Oct3/4 and Klf4 (Kim J B, et al.: Nature 454 (7204), 646-650, 2008), or Oct3/4 alone (Kim J B, et al.: Cell 136 (3), 411-419, 2009). In addition, a method for introducing a protein, which is an expression product of a gene, into cells (Zhou H, Wu S, Joo J Y, et al., Cell Stem Cell 4, 381-384, 2009, Kim D, Kim C H, Moon J I, et al., Cell Stem Cell 4, 472-476, 2009) has also been reported. On the other hand, it has also been reported that by using BIX-01294 which is an inhibitor of histone methyltransferase G9a, valproic acid (VPA) which is a histone deacetylase inhibitor, or Bay K8644, the production efficiency has been improved and the factors to be introduced have been reduced (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e253, 2008). Studies on gene transfer methods have also been carried out, and technologies for gene transfer have been developed using, in addition to a retrovirus, a lentivirus (Yu J, et al.: Science 318 (5858), 1917-1920, 2007), an adenovirus (Stadtfeld M, et al.: Science 322 (5903), 945-949, 2008), a plasmid (Okita K, et al.: Science 322 (5903), 949-953, 2008), a transposon vector (Woltjen K, Michael I P, Mohseni P, et al.: Nature 458, 766-770, 2009; Kaji K, Norrby K, Paca A, et al.: Nature 458, et al. 771-775, 2009; Yusa K, Rad R, Takeda J, et al.: Nat Methods 6, 363-369, 2009) or an episomal vector (Yu J, Hu K, Smuga-Otto K, Tian S, et al.: Science 324, 797-801, 2009).

Cells transformed to iPS cells, that is, cells that have undergone initialization (reprogramming) can be selected using, as an index, the expression of pluripotent stem cell markers (undifferentiated markers) such as Fbxo15, Nanog, Oct3/4, Fgf-4, Esg-1, and Cript, or the like. The selected cells can be recovered as iPS cells.

As the producing method of iPS cells, in addition to the method of manufacturing an iPS cell by direct initialization by gene expression, it is also possible to induce an iPS cell from a somatic cell by addition of a compound or the like (Hou P et al., Science 341(6146), 651-654, 2013).

The iPS cells can be provided from, for example, FUJIFILM Cellular Dynamics, Inc. (FCDI), National University Corporation Kyoto University, or Independent Administrative Institution RIKEN BioResource Center.

Examples of the method for obtaining nerve cells obtained by differentiation from a human-derived pluripotent stem cell, include inducing from human iPS cells produced from somatic cells collected from healthy humans (healthy person) who do not have a mutation of a disease-related gene causing a nervous system disease or who do not have a nervous system disease, or from somatic cells collected from patients who have a nervous system disease, and inducing from an established human iPS cell.

The nerve cells obtained by differentiation from a human-derived pluripotent stem cell, is not particularly limited, but is preferably a motor neuron, a cortical excitatory neuron, or a substantia nigra neuron, and particularly preferably the motor neuron or the cortical excitatory neuron.

A method for inducing differentiation into nerve cells from a human-derived pluripotent stem cell is not particularly limited, but includes a method of inducing differentiation into nerve cells after producing a neural stem cell from a pluripotent stem cell using a low-molecular-weight compound treatment or the like, and a method of directly inducing differentiation into nerve cells by gene expression or the like.

Examples of the method for inducing differentiation into nerve cells from a human-derived pluripotent stem cell include:
(1) a method of culturing the pluripotent stem cells in a serum-free medium to form embryoid bodies (cell aggregates including neuronal precursor cells) and then differentiating the pluripotent stem cells into nerve cells (SFEB method: Watanabe K., et al, Nat. Neurosci., 8: 288-296, 2005; SFEBq method: Wataya T., et al, Proc. Natl. Acad. Sci. USA., 105: 11796-11801, 2008);
(2) a method of culturing the pluripotent stem cells on stromal cells to differentiate the pluripotent stem cells into nerve cells (SDIA method: Kawasaki H., et al, Neuron, 28: 31-40, 2000);
(3) a method of culturing the pluripotent stem cells on a matrigel to which a drug has been added to differentiate the pluripotent stem cells into nerve cells (Chambers S. M., et al, Nat. Biotechnol., 27: 275-280, 2009);
(4) a method of culturing the pluripotent stem cells in a culture medium containing a low-molecular-weight compound as a substitute for a cytokine to differentiate the pluripotent stem cells into nerve cells (US5843780A);
(5) a method of introducing a neurogenic factor (neurogenin 2 (Ngn2) or the like) into the pluripotent stem cells and expressing the neurogenic factor to differentiate the pluripotent stem cells into nerve cells (WO2014/148646A; and Zhang Y., et al, Neuron, 78: 785-98, 2013);
(6) a method of introducing miR-9/9*-124 into the pluripotent stem cells and expressing miR-9/9*-124 to differentiate the pluripotent stem cells into nerve cells; and
   a combination of these methods.

Among the above, (5) the method of introducing neurogenin 2 into pluripotent stem cells and expressing the neurogenin 2 is preferable since mature nerve cells can be obtained in a short period of time with high efficiency.

The neurogenin 2 protein is a transcription factor known to promote differentiation into nerve cells during developmental stage, and the amino acid sequence thereof is exemplified as NP_076924 in humans and NP_033848 in mice. The neurogenin 2 gene (official full name: neurogenin 2, official symbol: NEUROG2, also referred to as Ngn2 gene) is DNA encoding a neurogenin 2 protein, and examples thereof include DNA having a nucleotide sequence of NM_009718 (mouse) or NM_024019 (human) registered as a standard sequence, or a transcript variant thereof.

In addition, the DNA may be a DNA having complementarity such that the DNA can be hybridized with the nucleic acid having the above-described standard sequence and the sequence of the transcriptional variant under stringent conditions.

As the cortical excitatory neuron obtained by differentiation from a human iPS cell, it is preferable to use a cell prepared by forcibly expressing the Ngn2 gene from a human iPS cell.

As the nerve cells obtained by differentiation from a human-derived pluripotent stem cell, a commercially available nerve cell may be used, and for example, iCell (trademark) motor neuron (FCDI, C1050, C1048) or the like may be used.

As the substantia nigra neuron obtained by differentiation from a human-derived pluripotent stem cell, a commercially available nerve cell may be used, and for example, iCell (trademark) dopamine nerve cells (FCDI, C1087, C1028) may be used.

The nerve cell is preferably a cell which expresses at least one or more marker genes specific to nerve cells consisting of β-III tubulin, NeuN, a neural cell adhesion molecule (N-CAM), and microtubule-associated protein 2 (MAP2), and has a β-III tubulin-positive protrusion (hereinafter, referred to as a neurite).

The expression level of a marker gene can be usually analyzed by the production amount of a transcript corresponding to the gene, or the production amount, the activity, and the like of a translation product thereof. The measurement of the expression level can be carried out by measuring an mRNA which is a transcript of a gene or a protein which is a translation product of a gene; however, it is preferably carried out by measuring an mRNA or a cDNA which is a reverse transcript thereof. The detection or measurement of the expression of the translation product (protein) can be performed by immunocytochemistry for detecting the protein in the cell using an antibody.

The culture of the nerve cells in the present invention may be performed by selecting a culture medium, a temperature, and other conditions according to the nerve cells to be used.

In the present invention, a culture medium that does not substantially contain an antioxidant and does not substantially contain an oxidant is used.

Examples of the antioxidant include vitamin A, glutathione, vitamin E or a derivative thereof, superoxide dismutase (SOD), catalase, and the like.

Examples of the oxidant include hydrogen peroxide, arsenite, sodium nitroprusside, and the like.

The term "substantially not contain" means that the antioxidant or the oxidant is not included in an amount at which the antioxidant or the oxidant exhibits its function.

Any component or additive such as a factor suitable for the purpose of culture may be contained in the culture medium as long as the culture of nerve cells is not disturbed.

A medium can be selected from the known media and commercially available media. The culture medium used for the culture can be used by adding an additive to the basal medium. Here, examples of the basal medium include DMEM, DMEM (Dulbecco's modified Eagle's medium)/F12, BrainPhys Neuronal Medium, Neurobasal Medium-A, Neurobasal Medium, Neural Progenitor Basal Medium, NS-A Basal Medium, Basal Medium Eagle (BME), BGJb Medium, CMRL 1066 Medium, Glasgow Minimum Essential Medium (MEM), Improved MEM Zinc Option, Iscove's Modified Dulbecco's Medium (IMDM), Medium 199, Eagle MEM, αMEM, Ham's F12 Medium, RPMI 1640 Medium, Fischer's Medium, and the like. DMEM/F12 or BrainPhys Neuronal Medium is preferable, and DMEM/F12 is more preferable. In addition, as the culture medium, a single culture medium may be used, or two or more culture media may be used in combination.

The additive may be added as long as it does not substantially exhibit an antioxidant action or may be added at a concentration at which it does not substantially exhibit an antioxidant action. Specific examples thereof include serum, retinoic acid, Wnt, BMP, bFGF, EGF, HGF, Sonic hedgehog (Shh), interleukins, heparin, heparan sulfate, collagen, fibronectin, progesterone, selenite, B-27 (trade name) supplement (containing no oxidation inhibitor), ITS-supplement, and the like, but the present invention is not particularly limited thereto. A preferred additive is B-27 (trademark) supplement (containing no oxidation inhibitor).

As the culture conditions for the nerve cell, general cell culture conditions may be selected. For example, conditions of 37°C and 5% CO₂.are mentioned. During the culture, the culture medium may be replaced at appropriate intervals (preferably once a day to 7 days and more preferably once every 2 days to 3 days), but it is preferable that the culture medium is not replaced during the culture period.

It is preferable that the nerve cells are subjected to two-dimensional culture. For culturing the cells, cell culture containers such as plates, dishes, and cell culture inserts, cell culture flasks can be used.

The nerve cells damaged by oxidative stress, which is produced by the production method of nerve cells according to the embodiment of the present invention, preferably satisfy at least one or more (preferably two and more preferably three) of the following (i) to (iv):
(i) a marker related to the oxidative stress is positive,
(ii) neurites are retracted,
(iii) necroptosis is induced, and
(iv) ferroptosis is induced.

According to the present invention, there is provided nerve cells, which is produced by the production method of nerve cells according to the embodiment of the present invention and satisfy at least one or more of the above-described (i) to (iv). The nerve cells according to the embodiment of the present invention are not particularly limited, but are preferably motor neurons, cortical excitatory neurons, or substantia nigra neurons, and more preferably motor neurons or cortical excitatory neurons.

Examples of the marker related to oxidative stress include an oxidative stress marker and a secondary marker caused by oxidative stress.

Examples of the oxidative stress marker include reactive oxygen species (ROS), an in vivo product generated by active oxygen, an antioxidant enzyme, an antioxidant substance, and the like. Examples of the ROS include a superoxide anion radical (O₂·⁻), hydrogen peroxide (H₂O₂), a hydroxyl radical (·OH), singlet oxygen (¹O₂), nitric oxide (NO·), nitrogen dioxide (NO₂·), ozone (O₃), a lipid peroxide (LOOH), and the like. Examples of the in vivo product generated by active oxygen include hydroxydeoxyguanosine (8-OHdG), 8-hydroxyguanosine (8-OHG), lipid peroxide, and the like. Examples of the antioxidant enzyme include SOD, catalase, GPx, and the like. Examples of the antioxidant substance include glutathione (GSH/GSSG), bilirubin, vitamins, and the like.

Examples of the secondary marker caused by oxidative stress include phosphorylation, accumulation, and aggregation of TAR DNA binding protein 43 (TDP-43) protein, amyloid β protein, tau protein, and α-synuclein.

As the marker related to oxidative stress, ROS, 8-OHdG, 8-OHG, lipid peroxide, glutathione, TDP-43 protein, amyloid β protein, tau protein, or α-synuclein is preferable, and ROS is more preferable.

The fact that the reactive oxygen species (ROS) are positive indicates that the level of ROS is high as compared with the level of ROS in cells cultured using a culture medium containing an antioxidant. The level of ROS in the cell can be quantified using a commercially available reagent such as CellROX (trademark) Green Reagent, for oxidative stress detection (Thermo Fisher Scientific, C10444).

The retraction of the neurites can be confirmed by measuring the neurite length. For example, the neurite length can be quantified using the Neurotrack software (Sartorius, 9600-0010) of IncuCyte S3.

Whether or not necroptosis is induced can be determined, for example, by evaluating the degree of damage due to oxidative stress between a case where cells are treated with a necroptosis inhibitor (for example, Necrostatin-1) and a case where cells are not treated with a necroptosis inhibitor (for example, Necrostatin-1), and in a case where the degree of the damage is reduced in the case where cells are treated with a necroptosis inhibitor (for example, Necrostatin-1), it can be determined that necroptosis is induced. In addition, it is also possible to determine whether or not necroptosis is induced by quantifying the phosphorylation of receptor interacting protein kinase 1 (RIPK1), receptor interacting protein kinase 3 (RIPK3), Mixed lineage kinase domain-like protein (MLKL), and the like, which are known as markers related to necroptosis.

Whether or not ferroptosis is induced can be determined by, for example, evaluating the degree of damage due to oxidative stress between a case where cells are treated with a ferroptosis inhibitor (for example, Ferrostatin-1, Liproxstatin-1, UAMC-3203, or the like) and a case where cells are not treated with a ferroptosis inhibitor (for example, Ferrostatin-1, Liproxstatin-1, UAMC-3203, or the like), and in a case where the degree of damage is reduced in a case where cells are treated with a ferroptosis inhibitor (for example, Ferrostatin-1, Liproxstatin-1, UAMC-3203, or the like), it can be determined that ferroptosis is induced. In addition, it is also possible to determine whether or not ferroptosis is induced by quantifying the intracellular amount of divalent iron or the lipid peroxide (4-hydroxynonanal or malondialdehyde), which is known as a marker related to ferroptosis.

According to the present invention, it is possible to produce nerve cells damaged by oxidative stress. The evaluation of whether or not the nerve damage due to oxidative stress is received can be performed by evaluating cell death (the number of nerve cells), measuring a marker related to oxidative stress, evaluating cellular damage, measuring a marker related to nerve damage, or measuring the neurite length, but is not particularly limited. Among the above, it is preferable to evaluate cell death (the number of nerve cells) or to measure the neurite length.

The cell death can be detected by using a live cell detection reagent such as Cell Titer Glo (Promega) or Cell counting kit-8 (Dojindo), or by using a cell death detection reagent such as Propidium Iodide or NucGreen Dead (Thermo Fisher Scientific).

The cellular damage can be evaluated by using an evaluation method such as an LDH assay, a WST assay, or an ATP assay. For example, a kit such as Cytotoxicity Detection KitPLUS (LDH) (Sigma Aldrich) can be used for the LDH assay.

Examples of the marker related to the nerve damage include Enolase 2 (neuron-specific enolase), and the measurement can be performed using an ELISA method.

The nerve cells damaged by oxidative stress according to the present invention can be used for screening a new drug useful for neurodegenerative diseases in which oxidative stress is known to be significantly involved in the onset and progression. The nerve cell damaged by oxidative stress according to the present invention is particularly useful for research on sporadic chronic neurodegenerative diseases, the development of therapeutic drugs for the diseases, and the identification of disease biomarkers and the development of diagnostic drugs. In the present invention, it is preferable to use nerve cells produced from a pluripotent stem cell having no mutation in a disease-related gene, and to evaluate an sporadic chronic neurodegenerative disease by using a culture medium that does not substantially contain an antioxidant and does not substantially contain an oxidant, and by chronically applying weak oxidative stress to cells.

In the present invention, since cell death is observed after 2 days from the start of culture, the drug can be evaluated in about 2 weeks. Furthermore, in the present invention, since cell death due to necroptosis can be reproduced, screening of a necroptosis inhibitor can be performed. In addition, in the present invention, since cell death due to ferroptosis can be reproduced, screening of a ferroptosis inhibitor can be performed.

That is, according to the present invention, there is provided an evaluation method for a test substance, which includes bringing a test substance into contact with nerve cells damaged by oxidative stress according to the present invention.

According to the present invention, there is provided a screening method for a drug for prevention and/or treatment of a neurodegenerative disease, the method including bringing a test substance into contact with nerve cells according to the present invention.

According to the present invention, there is further provided a screening method for a necroptosis inhibitor, including:
(1) a step of bringing a test substance into contact with nerve cells according to the present invention,
(2) a step of culturing the nerve cells that has been brought into contact with the test substance in the step (1) and nerve cells, used as a control, that have never been brought into contact with the test substance,
(3) a step of measuring nerve damage in the nerve cells, and
(4) a step of selecting, as a candidate for a necroptosis inhibitor, a test substance that suppresses the nerve damage in comparison with the nerve cells, used as a control, that have never been brought into contact with the test substance.

According to the present invention, there is further provided a screening method for a ferroptosis inhibitor, including:
(1) a step of bringing a test substance into contact with nerve cells according to the present invention,
(2) a step of culturing the nerve cells that has been brought into contact with the test substance in the step (1) and nerve cells, used as a control, that have never been brought into contact with the test substance,
(3) a step of measuring nerve damage in the nerve cells, and
(4) a step of selecting, as a candidate for a ferroptosis inhibitor, a test substance that suppresses the nerve damage in comparison with the nerve cells, used as a control, that have never been brought into contact with the test substance.

The above-described evaluation method for a test substance, screening method for a drug for prevention and/or treatment of a neurodegenerative disease, screening method for a necroptosis inhibitor, and screening method for a ferroptosis inhibitor may further include producing the nerve cells according to the embodiment of the present invention by the production method of nerve cells damaged by oxidative stress according to the embodiment of the present invention.

Examples of the test substance include a protein, a peptide, an antibody, a nucleic acid (a gene expression vector, siRNA, an antisense oligonucleotide, or mRNA), a virus vector (AAV, lentivirus, adenovirus, or the like), a non-peptidic compound, a synthetic compound, a synthetic low-molecular-weight compound, a natural compound, a cell extract, an extracellular vesicle, a plant extract, an animal tissue extract, a blood plasma, an extract derived from a marine organism, a cell culture supernatant, a microbial fermentation product, and the like.

In addition, the test substance can be obtained by using any of a plurality of approaches in a combinatorial library method known in the related art, including (1) biological library method, (2) synthetic library method using deconvolution, (3) one-bead one-compound library method, and (4) synthetic library method using affinity chromatography sorting. The biological library method using affinity chromatography sorting is limited to a peptide library, but other approaches can be applied to a low-molecular-weight compound library of peptides, non-peptide oligomers, or compounds (Lam (1997) Anticancer Drug Des. 12: 145-67). Examples of the synthesis method of the molecular library can be found in the related art (DeWitt et al. (1993) Proc.Natl.Acad.Sci.USA 90: 6909-13; Erb et al. (1994) Proc.Natl.Acad.Sci.USA91: 11422-6; Zuckermann et al. (1994) J.Med.Chem. 37: 2678-85; Cho et al. (1993) Science 261: 1303-5; Carell et al. (1994) Angew.Chem.Int.Ed.Engl. 33: 2059; Carell et.al. (1994) Angew.Chem.Int.Ed.Engl. 33: 2061; Gallop et al. (1994) J.Med.Chem. 37: 1233-51). The compound library can be prepared as a solution (see Houghten (1992) Bio/Techniques 13: 412-21), beads (Lam (1991) Nature 354: 82-4), chips (Fodor (1993) Nature 364: 555-6), bacteria (US5223409A), spores (US5571698A, US5403484A, and US5223409A), plasmids (Cull et al. (1992) Proc. Natl. Acad. Sci. USA 89: 1865-9), or phages (Scott and Smith (1990) Science 249: 386-90; Devlin (1990) Science 249: 404-6; Cwirla et al. (1990) Proc. Natl. Acad. Sci. USA 87: 6378-82; and Felici (1991) J. Mol. Biol. 222: 301-10; US2002/0103360A).

Bringing the test substance into contact with the nerve cells may be performed by adding the test substance to a culture solution of the nerve cells. The contact is not particularly limited as long as the change in the index can be confirmed, but is, for example, 1 day or more, 2 days or more, 3 days or more, 4 days or more, 5 days or more, 6 days or more, or 7 days or more. The concentration of the test substance to be added can be appropriately adjusted depending on the type of the compound (solubility, toxicity, and the like).

The culture solution of nerve cells, which is used in a case of bringing a test substance into contact with nerve cells, is not particularly limited as long as it is a culture medium in which nerve cells can be cultured.

The culture temperature in a case where the test substance is brought into contact with the nerve cells is not particularly limited, but is approximately 30°C to 40°C and preferably about 37°C, and the culture is performed in an atmosphere of CO₂-containing air, and the CO₂ concentration is preferably about 2% to 5%.

Examples of the neurodegenerative disease include Alzheimer's disease (AD), spinocerebellar degeneration, frontotemporal lobar degeneration (FTLD), Parkinson's disease, amyotrophic lateral sclerosis (ALS), dementia with Lewy bodies, Huntington's disease, Niemann-Pick disease, and the like. As the neurodegenerative disease, Alzheimer's disease (AD), Parkinson's disease, amyotrophic lateral sclerosis (ALS), Huntington's disease, or Niemann-Pick disease is preferable, and amyotrophic lateral sclerosis (ALS) is more preferable.

Examples of diseases in which necroptosis is involved include neurodegenerative diseases, acute kidney injury, alcoholic liver injury, non-alcoholic fatty liver disease, non-alcoholic steatohepatitis, acute lung injury, acute respiratory distress syndrome, systemic inflammatory response syndrome, chronic obstructive pulmonary disease, idiopathic pulmonary fibrosis, heart failure, arteriosclerosis, aortic aneurysm, psoriasis, rheumatoid arthritis, acute myeloid leukemia, chronic lymphocytic leukemia, head and neck squamous cell carcinoma, non-small cell lung cancer, ovarian cancer, colon cancer, cervical cancer, malignant melanoma, glioblastoma, lung cancer, breast cancer, pancreatic cancer, and the like. As the disease involved in necroptosis, a neurodegenerative disease is preferable, multiple sclerosis (MS), amyotrophic lateral sclerosis (ALS), Alzheimer's disease (AD), and Parkinson's disease (PD) are preferable, and amyotrophic lateral sclerosis (ALS) is more preferable.

Examples of diseases involved in ferroptosis include neurodegenerative diseases, age-related macular degeneration, Fuchs' endothelial corneal dystrophy, chronic obstructive pulmonary disease, radiation lung injury, acute lung injury, asthma, pulmonary fibrosis, tuberculosis, Pseudomonas aeruginosa infection, paraquat poisoning, ischemia-reperfusion injury, alcoholic liver injury, autoimmune hepatitis, non-alcoholic steatohepatitis, acetaminophen-induced liver injury, liver fibrosis, liver transplantation, acute pancreatitis, diabetes, islet transplantation, hemochromatosis, transfusion-related immunomodulation, hemolytic anemia, radiation-induced hematopoietic injury, periventricular leukomalacia, hemorrhagic/ischemic stroke, hemorrhagic dementia, traumatic brain injury, epilepsy, ischemia-reperfusion injury, doxorubicin cardiomyopathy, iron overload cardiomyopathy, myocardial infarction/fibrosis, atherosclerosis, heart transplantation, acute kidney injury, polycystic kidney disease, kidney transplantation, Crohn's disease, ulcerative colitis, hypertensive nephropathy associated with pregnancy, endometriosis, infertility, neuroblastoma, glioblastoma, colon cancer, lung cancer, head and neck cancer, gastric cancer, pancreatic adenocarcinoma, breast cancer, ovarian cancer, hepatocellular carcinoma, renal cell carcinoma, Burkitt's lymphoma, and the like. As the disease involved in ferroptosis, a neurodegenerative disease is preferable, Alzheimer's disease (AD), Parkinson's disease (PD), amyotrophic lateral sclerosis (ALS), Huntington's disease, or Niemann-Pick disease is preferable, and amyotrophic lateral sclerosis (ALS) is more preferable.

In one aspect of the present invention, it is possible to perform:
after a test substance is brought into contact with the nerve cells according to the embodiment of the present invention,
(A) a step of culturing the nerve cells that has been brought into contact with the test substance and nerve cells, used as a control, that have never been brought into contact with the test substance,
(B) a step of measuring nerve damage in the nerve cells, and
(C) a step of selecting, as a candidate for a drug for prevention and/or treatment of a neurodegenerative disease, a test substance that suppresses the nerve damage in comparison with the nerve cells, used as a control, that have never been brought into contact with the test substance.

The step (B) is preferably a step of measuring the number and/or the neurite length of the nerve cells obtained in the step (A).

The step (C) is preferably a step of selecting, as a candidate for a drug for prevention and/or treatment of a neurodegenerative disease, a test substance that has been brought into contact with the nerve cells and causes the number and/or the neurite length of the nerve cells to be greater than that in the control.

The present invention will be more specifically described with reference to Examples, but the present invention is not limited to the scope of Examples.

### Examples

Testing Example 1: Evaluation of neurite length of nerve cells cultured in culture medium not containing antioxidant from time of cell seeding and nerve cells cultured in culture medium not containing antioxidant replaced by culture medium exchange after cell seeding

### <Plate coating>

A solution obtained by diluting iMatrix-511 silk (Matrixome, 892021) 16.7 times with phosphate buffered saline (PBS) was added to a 96-well plate (Corning, 356461) coated with poly-D-lysine (PDL) at 70 µL/well, and incubated at 37°C for 3 hours to 72 hours.

### <Preparation of culture medium>

As a culture medium containing an antioxidant, a culture medium (Stress(-)) in which dimethyl sulfoxide (DMSO) was added to the +AO culture medium to a final concentration of 0.1% by volume (v/v) was used, and as a culture medium not containing an antioxidant, a culture medium (Stress(+)) in which DMSO was added to the -AO culture medium to a final concentration of 0.1% by volume (v/v) was used.

**[Table 1]**

| +AO culture medium | |
|---|---|
| | mL |
| DMEM/F12(Thermo Fisher Scientific, 11320-033) | 4.9 |
| B-27 Supplement (50X), serum free (Thermo Fisher Scientific, 17504-044) | 0.1 |

| -AO culture medium | |
|---|---|
| | mL |
| DMEM/F12(Thermo Fisher Scientific, 11320-033) | 9.8 |
| B-27 Supplement (50X), minus antioxidants (Thermo Fisher Scientific. 10889-038) | 0.2 |

### <Cell seeding>

Nerve cells (considered to be cortical excitatory neurons) prepared by forcibly expressing a neurogenin 2 gene (Ngn2 gene) from iPS cells (Chao Wang., et al., Stem Cell Reports., 9: 1221-1233, 2017) was thawed in a warm bath at 37°C. After melting, the cells were added to a culture medium and centrifuged at 600 × g at room temperature for 5 minutes. After centrifugation, the supernatant was removed, the cells were suspended in a culture medium, and the number of cells was counted. Thereafter, the cells were diluted with a culture medium, seeded at 200 µL/well (9.4 × 10⁴ cells/cm²), and cultured under the conditions of 37°C and 5% CO₂. Imaging was performed every 6 hours with IncuCyte S3 (Sartorius, Incucyte S3) to acquire images. After culturing, the culture medium was exchanged 48, 72, or 96 hours later.

### <Evaluation of neurite length>

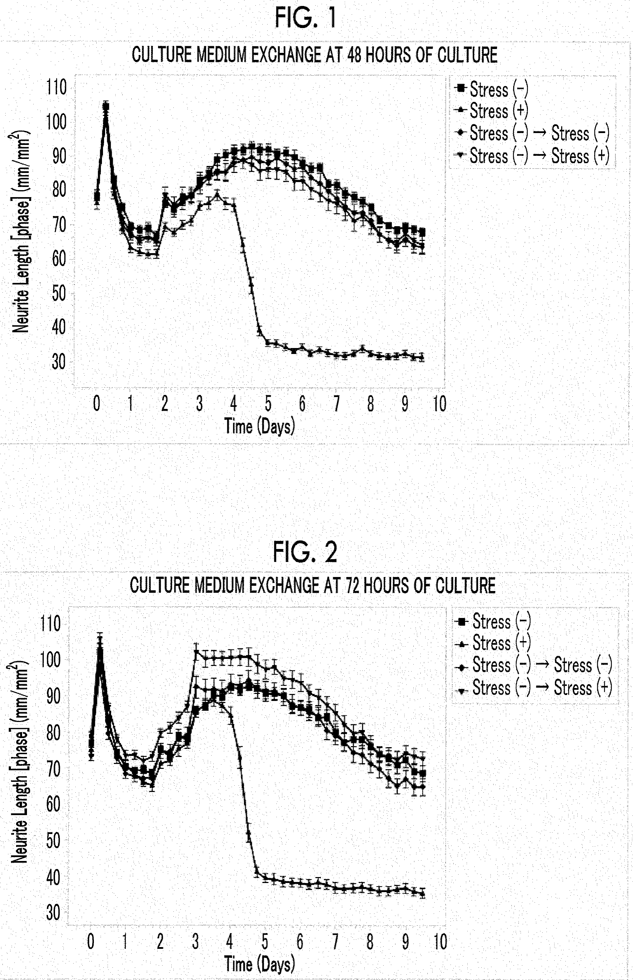

The neurite length was quantified using the Neurotrack software (Sartorius, 9600-0010) of IncuCyte S3. In a case where, under Stress(+) condition cultured in culture medium not containing an antioxidant, at any time point after neurite retraction and by 14th day of culture, the neurite length (the sum of neurite lengths per area) was equal to or less than half of the maximum neurite length observed in the Stress(-) group cultured in culture medium containing an antioxidant, the neurite was determined to be retracted. The results of quantifying the neurite length are shown in Figs. 1 to 3.

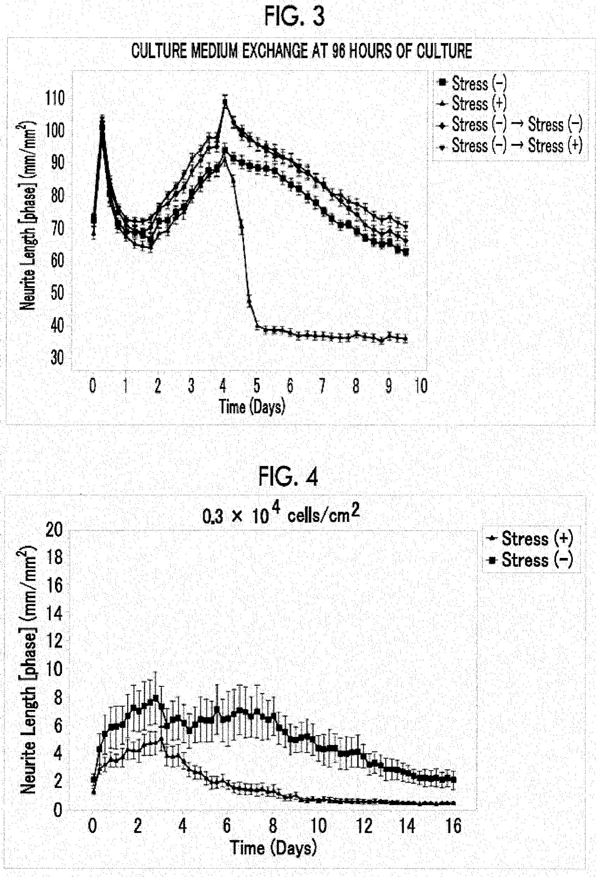

Fig. 1 is a result of quantifying the neurite length of the nerve cells with the culture medium exchanged at 48 hours of culture. The vertical axis represents the sum of neurite lengths per area, and the horizontal axis represents the number of days of culture. As a result, in the Stress(+) group (A) cultured in a culture medium not containing an antioxidant from the beginning, the retraction of the neurites (nerve damage) was confirmed after 4 days of culture. On the other hand, in the Stress(-) group (▪) cultured in a culture medium containing an antioxidant from the beginning, the group ( ◆ ) that was initially cultured in a culture medium containing an antioxidant and then subjected to culture medium exchange with a culture medium containing the same antioxidant after 48 hours, and the group (▼) that was initially cultured in a culture medium containing an antioxidant and then subjected to culture medium exchange with a culture medium not containing an antioxidant after 48 hours, the retraction of the neurites (nerve damage) was not confirmed. In any of the cells in which the culture medium was exchanged after 72 hours of culture (Fig. 2) or the cells in which the culture medium was exchanged after 96 hours of culture (Fig. 3), the retraction of the neurites (nerve damage) was confirmed only in the Stress(+) group (A) cultured in the culture medium containing no antioxidant from the beginning, but the retraction of the neurites (nerve damage) was not confirmed in the Stress(-) group (▪) cultured in the culture medium containing an antioxidant from the beginning, the group (◆) that was initially cultured in the culture medium containing an antioxidant and then subjected to the culture medium exchange in the same culture medium, and the group (▼) that was initially cultured in the culture medium containing an antioxidant and then subjected to the culture medium exchange with the culture medium containing no antioxidant. From these facts, it was shown that to induce nerve damage under weak oxidative stress (culture in a culture medium not containing an antioxidant), culture in a culture medium not containing an antioxidant from time of cell seeding.

### Test Example 2: Evaluation of effect of cell seeding density on oxidative stress-induced neuronal damage

### <Plate coating>

A solution obtained by diluting iMatrix-511 silk (Matrixome, 892021) 16.7 times with PBS was added to a 96-well plate (Corning, 356461) coated with PDL at 70 µL/well, and incubated at 37°C for 3 hours to 72 hours.

### <Preparation of culture medium>

As a culture medium containing an antioxidant, a culture medium (Stress(-)) in which DMSO was added to a +AO culture medium to a final concentration of 0.1% by volume (v/v) was used, and as a culture medium not containing an antioxidant, a culture medium (Stress(+)) in which DMSO was added to a -AO culture medium to a final concentration of 0.1% by volume (v/v) was used.

**[Table 2]**

| +AO culture medium | |
|---|---|
| | mL |
| DMEM/F12 (Thermo Fisher Scientific, 11320-033) | 4.9 |
| B-27 Supplement (50X), serum free (Thermo Fisher Scientific, 17504-044) | 0.1 |

| -AO culture medium | |
|---|---|
| | mL |
| DMEM/F12 (Thermo Fisher Scientific, 11320-033) | 9.8 |
| B-27 Supplement (50X), minus antioxidants (Thermo Fisher Scientific. 10889-038) | 0.2 |

### <Cell seeding>

The nerve cells (considered to be cortical excitatory neurons) produced by forcibly expressing the Ngn2 genes from the iPS cells in the same manner as in Test Example 1 was thawed in a warm bath at 37°C. After melting, the cells were added to a culture medium and centrifuged at 600 × g at room temperature for 5 minutes. After centrifugation, the supernatant was removed, the cells were suspended in a culture medium, and the number of cells was counted. Thereafter, the cells were diluted with a culture medium, seeded at 200 µL/well (0.3 × 10⁴ to 37.5 × 10⁴ cells/cm²), and cultured under the conditions of 37°C and 5% CO₂. After culturing, images were acquired by imaging with IncuCyte S3 (Sartorius, Incucyte S3) every 6 hours.

### <Evaluation of neurite length>

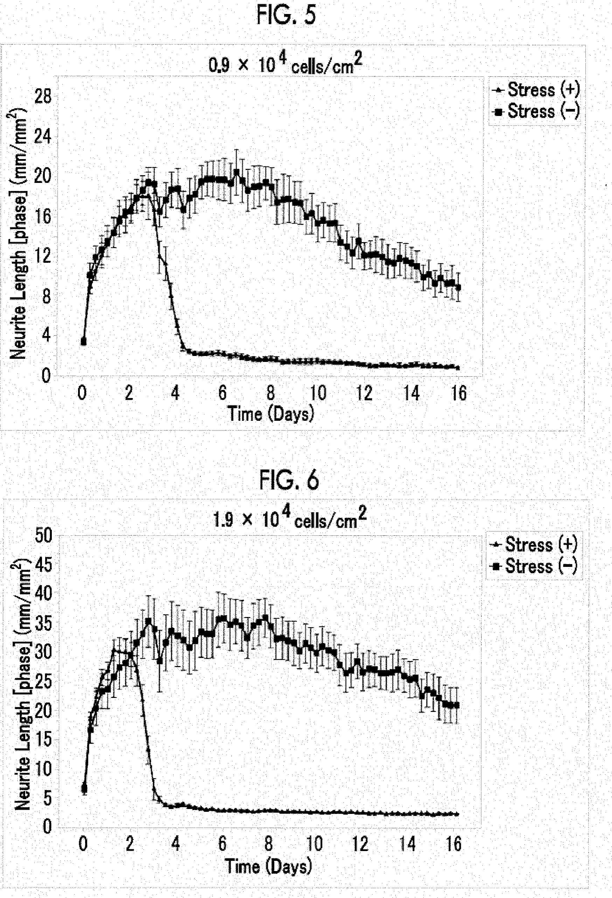

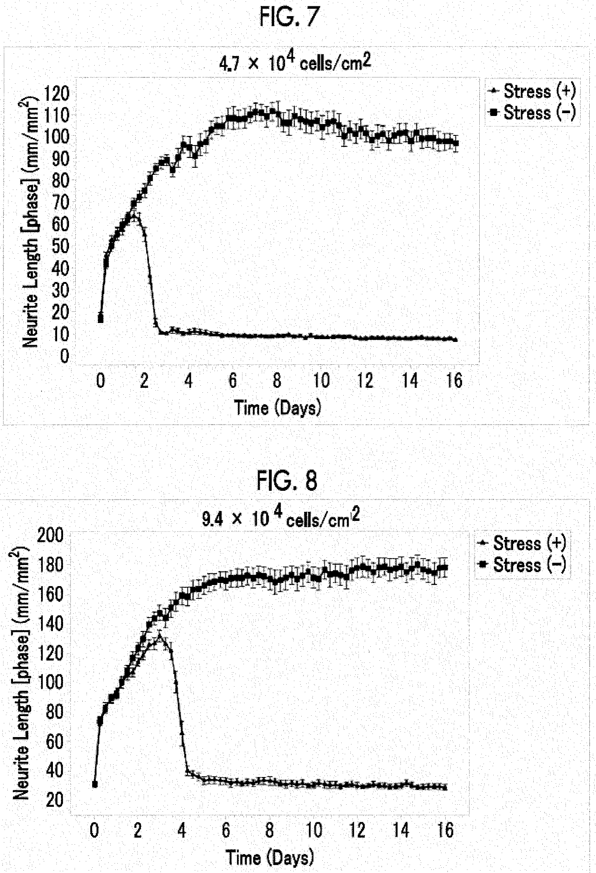

The neurite length was quantified using the Neurotrack software (Sartorius, 9600-0010) of IncuCyte S3. The results of the quantification of the neurite length are shown in Figs. 4 to 9, and the summary of the cell seeding density and the possibility of damage detection of the nerve cells is shown in Table 3. The possibility of the damage detection of the nerve cells was evaluated based on the same determination criteria as in Test Example 1, and in a case where it was determined that the neurite length was retracted, it was determined that the damage was detectable.

**[Table 3]**

| Cell seeding density (×10⁴ cells/cm²) | Possibility of damage detection |
|---|---|
| 0.3 | Damage detectable |
| 0.9 | Damage detectable |
| 1.9 | Damage detectable |
| 3.1 | Damage detectable |
| 4.7 | Damage detectable |
| 9.4 | Damage detectable |
| 10.9 | Damage detectable |
| 12.5 | Damage detectable |
| 25.0 | Damage not detectable |
| 37.5 | Damage not detectable |

Figs. 4 to 9 show changes over time in the neurite length in a case where the cells are seeded at a cell seeding density of 0.3, 0.9, 1.9, 4.7, 9.4, or 12.5 × 10⁴ cells/cm². The vertical axis represents the sum of neurite lengths per area, and the horizontal axis represents the number of days of culture. As a result, in the Stress(+) group (A) cultured in the culture medium containing no antioxidant at a cell seeding density of 0.3 to 12.5 cells/cm², the retraction of the neurites (nerve damage) was confirmed. The experimental results obtained by seeding at a seeding density of 0.3 × 10⁴ to 37.5 × 10⁴ cells/cm² are shown in Table 3. In a case where the seeding density was 25.0 × 10⁴ cells/cm² or more, since the cell density was too high, the retraction of the neurite (nerve damage) could not be detected. From this, it has been clarified that it is important to seed and culture cells at a cell density of 20.0 × 10⁴ cells/cm² or less.

### Test Example 3: Evaluation of effect of basal medium on oxidative stress-induced neuronal damage

### <Plate coating>

A solution obtained by diluting iMatrix-511 silk (Matrixome, 892021) 16.7 times with PBS was added to a 96-well plate (Corning, 356461) coated with PDL at 70 µL/well, and incubated at 37°C for 3 hours to 72 hours.

### <Preparation of culture medium>

As a culture medium containing an antioxidant, a culture medium (Stress(-)) in which DMSO was added to a +AO culture medium to a final concentration of 0.1% by volume (v/v) was used, and as a culture medium not containing an antioxidant, a culture medium (Stress(+)) in which DMSO was added to a -AO culture medium to a final concentration of 0.1% by volume (v/v) was used.

**[Table 4]**

| +AO culture medium | |
|---|---|
| | mL |
| BrainPhys medium (Stemcell technologies, 05790), or Neurobasal medium (Thermo Fisher Scientific, 21103049) | 4.9 |
| B-27 Supplement (50X), serum free (Thermo Fisher Scientific, 17504-044) | 0.1 |

| -AO culture medium | |
|---|---|
| | mL |
| BrainPhys medium (Stemcell technologies, 05790), or Neurobasal medium (Thermo Fisher Scientific, 21103049) | 9.8 |
| B-27 Supplement (50X), minus antioxidants (Thermo Fisher Scientific, 10889-038) | 0.2 |

### <Cell seeding>

The nerve cells (considered to be cortical excitatory neurons) produced by forcibly expressing the Ngn2 genes from the iPS cells in the same manner as in Test Example 1 was thawed in a warm bath at 37°C. After melting, the cells were added to a culture medium and centrifuged at 600 × g at room temperature for 5 minutes. After centrifugation, the supernatant was removed, the cells were suspended in a culture medium, and the number of cells was counted. Thereafter, the cells were diluted with a culture medium, seeded at 200 µL/well (4.7 × 10⁴ cells/cm²), and cultured under the conditions of 37°C and 5% CO₂. After culturing, images were acquired by imaging with IncuCyte S3 (Sartorius, Incucyte S3) every 6 hours.

### <Evaluation of neurite length>

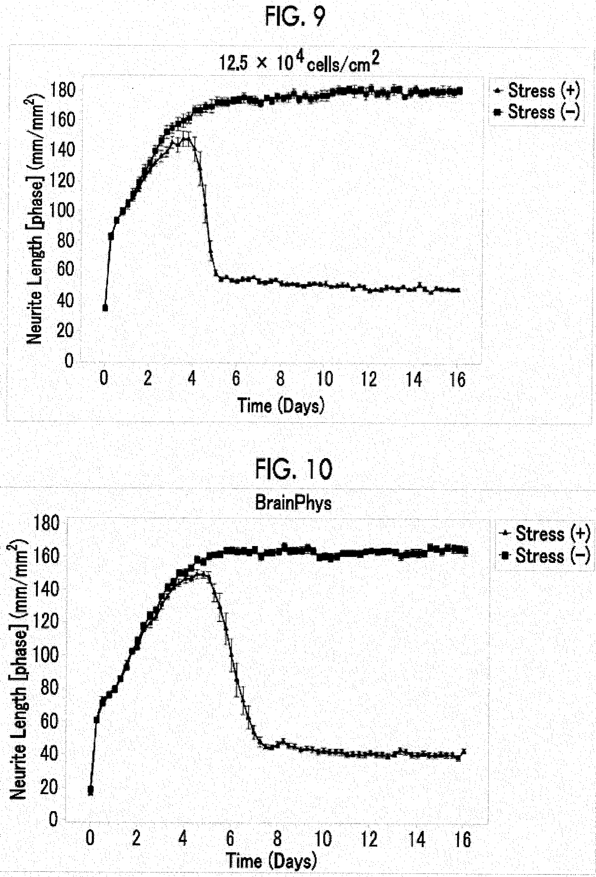

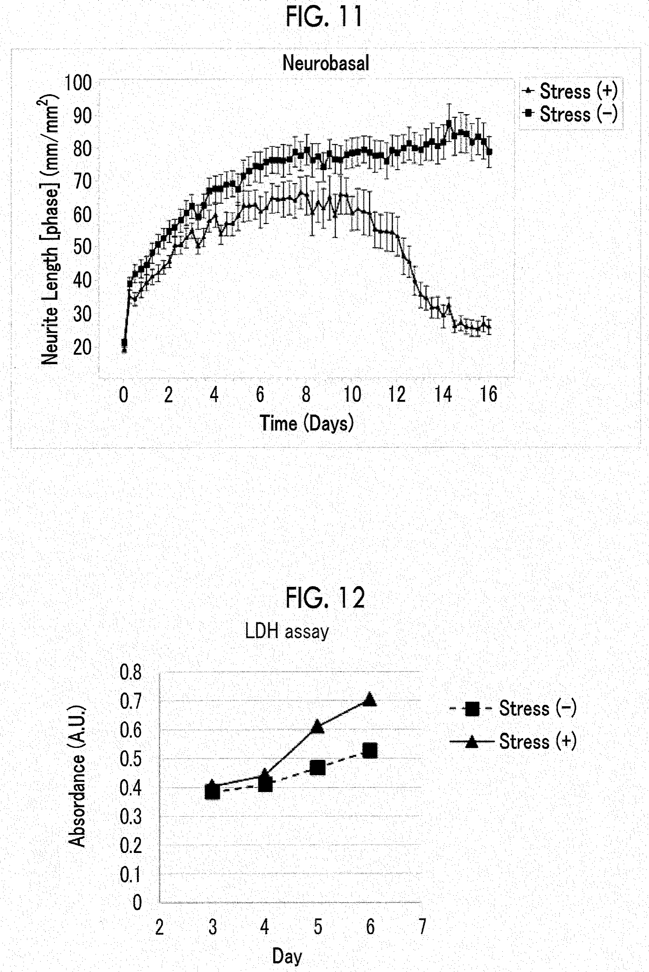

The neurite length was quantified using the Neurotrack software (Sartorius, 9600-0010) of IncuCyte S3. The results of quantifying the neurite length are shown in Figs. 10 and 11. The retraction of the neurite was evaluated according to the same determination criteria as in Test Example 1.

The vertical axis represents the sum of neurite lengths per area, and the horizontal axis represents the number of days of culture. As a result, in both the BrainPhys culture medium (Fig. 10) and the Neurobasal culture medium (Fig. 11), the retraction of the neurites (nerve damage) was confirmed in the Stress(+) group (A) cultured in the culture medium not containing an antioxidant, and the retraction of the neurites was not confirmed in the Stress(-) group (▪) cultured in the culture medium containing an antioxidant. From this, it was shown that the culture in a culture medium not containing an antioxidant is also capable of inducing nerve damage in the BrainPhys medium or the Neurobasal medium.

### Test Example 4: Quantitative evaluation of oxidative stress-induced neuronal damage

### <Plate coating>

A solution obtained by diluting iMatrix-511 silk (Matrixome, 892021) 16.7 times with PBS was added to flask (Greiner Bio-one, 661940) coated with PDL at 20 mL/flask, and incubated at 37°C for 3 hours to 72 hours.

### <Preparation of culture medium>

As a culture medium containing an antioxidant, a culture medium (Stress(-)) in which DMSO was added to a +AO culture medium to a final concentration of 0.1% by volume (v/v) was used, and as a culture medium not containing an antioxidant, a culture medium (Stress(+)) in which DMSO was added to a -AO culture medium to a final concentration of 0.1% by volume (v/v) was used.

**[Table 5]**

| +AO culture medium | |
|---|---|
| | mL |
| DMEM/F12 (Thermo Fisher Scientific, 11320-033) | 4.9 |
| B-27 Supplement (50X), serum free (Thermo Fisher Scientific, 17504-044) | 0.1 |

| -AO culture medium | |
|---|---|
| | mL |
| DMEM/F12 (Thermo Fisher Scientific, 11320-033) | 29.4 |
| B-27 Supplement (50X), minus antioxidants (Thermo Fisher Scientific. 10889-038) | 0.6 |

### <Cell seeding>

The nerve cells (considered to be cortical excitatory neurons) produced by forcibly expressing the Ngn2 genes from the iPS cells in the same manner as in Test Example 1 was thawed in a warm bath at 37°C. After melting, the cells were added to a culture medium and centrifuged at 600 × g at room temperature for 5 minutes. After centrifugation, the supernatant was removed, the cells were suspended in 1 mL of the culture medium, and the number of cells was counted. Thereafter, the cells were diluted with a culture medium, seeded to a density of 6.9 × 10⁴ cells/cm², and cultured under the conditions of 37°C and 5% CO₂. On the 3rd day, the 4th day, the 5th day, and the 6th day after the culturing, the culture medium was collected.

### <LDH assay>

For the quantification of LDH, Cytotoxicity Detection KitPLUS (LDH) (Sigma aldrich, 4744934001) was used. The quantification of LDH in the culture medium from the 3rd day to the 6th day of culture was performed according to the attached document. The results of the quantification are shown in Fig. 12.

In the culture medium of the nerve cells in the Stress(+) group (**▲**), which were cultured in the culture medium not containing an antioxidant, the amount of LDH was larger on the 5th and 6th days of culture than that in the culture medium of the nerve cells in the Stress(-) group (▪), which were cultured in the culture medium containing an antioxidant, and it was confirmed that cellular damage occurred in the Stress(+) group.

### Test Example 5: Quantification of ROS signal

### <Plate coating>

A solution obtained by diluting iMatrix-511 silk (Matrixome, 892021) 16.7 times with PBS was added to a 96-well plate (Corning, 356640) coated with PDL at 70 µL/well, and incubated at 37°C for 3 hours to 72 hours.

### <Preparation of culture medium>

As a culture medium containing an antioxidant, a culture medium (Stress(-)) in which DMSO was added to a +AO culture medium to a final concentration of 0.1% by volume (v/v) was used, and as a culture medium not containing an antioxidant, a culture medium (Stress(+)) in which DMSO was added to a -AO culture medium to a final concentration of 0.1% by volume (v/v) was used.

**[Table 6]**

| +AO culture medium | |
|---|---|
| | mL |
| DMEM/F12 (Thermo Fisher Scientific, 11320-033) | 4.9 |
| B-27 Supplement (50X), serum free (Thermo Fisher Scientific, 17504-044) | 0.1 |

| -AO culture medium | |
|---|---|
| | mL |
| DMEM/F12 (Thermo Fisher Scientific, 11320-033) | 9.8 |
| B-27 Supplement (50X), minus antioxidants (Thermo Fisher Scientific. 10889-038) | 0.2 |

### <Cell seeding>

Frozen cells (iCell motor neurons, FCDI, C1048) were thawed in a warm bath at 37°C. After melting, the cells were added to a culture medium and centrifuged at 600 × g at room temperature for 5 minutes. After centrifugation, the supernatant was removed, the cells were suspended in 1 mL of the culture medium, and the number of cells was counted. Thereafter, the cells were diluted with a culture medium, seeded at 200 µL/well (4.7 × 10⁴ cells/cm²), and cultured under the conditions of 37°C and 5% CO₂ for 6 days.

### <Quantification of ROS signal>

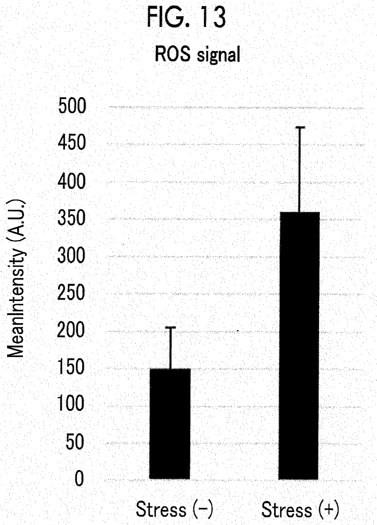

CellROX (trademark) Green Reagent, for oxidative stress detection (Thermo Fisher Scientific, C10444) was used for the quantification of the ROS signal. The quantification was performed according to the attached document, and the intracellular ROS signal of the nerve cells after 6 days of culture was quantified using a confocal quantitative image cytometer (CQ1, Yokogawa Electric Corporation). Fig. 13 shows the results of quantifying the average of the fluorescence intensity of the nuclear region.

In the nerve cells (Stress(+) group) cultured in the culture medium not containing an antioxidant, a stronger signal of ROS was detected as compared with the nerve cells (Stress(-) group) cultured in the culture medium containing an antioxidant.

### Test Example 6: Evaluation of compound (necroptosis inhibitor) for oxidative stress-induced neuronal damage

### <Plate coating>

A solution obtained by diluting iMatrix-511 silk (Matrixome, 892021) 16.7 times with PBS was added to a 96-well plate (Corning, 356461) coated with PDL at 70 µL/well, and incubated at 37°C for 3 hours to 72 hours.

### <Preparation of assay plate>

As a culture medium containing an antioxidant, a culture medium (Stress(-)) in which DMSO was added to a +AO culture medium to a final concentration of 0.1% by volume (v/v) was used, as a culture medium not containing an antioxidant, a culture medium (Stress(+)) in which DMSO was added to a -AO culture medium to a final concentration of 0.1% by volume (v/v), and as the culture medium of the drug evaluation group, a culture medium in which a DMSO-dissolved compound was added to the -AO culture medium to a concentration of 0.1% by volume (v/v) (0.01 to 20 µM) was used.

**[Table 7]**

| +AO culture medium | |
|---|---|
| | mL |
| DMEM/F12 (Thermo Fisher Scientific, 11320-033) | 4.9 |
| B-27 Supplement (50X), serum free (Thermo Fisher Scientific, 17504-044) | 0.1 |

| -AO culture medium | |
|---|---|
| | mL |
| DMEM/F12 (Thermo Fisher Scientific, 11320-033) | 9.8 |
| B-27 Supplement (50X), minus antioxidants (Thermo Fisher Scientific. 10889-038) | 0.2 |

### <Cell seeding>

Frozen cells (iCell motor neurons, FCDI, C1048) were thawed in a warm bath at 37°C. After melting, the cells were added to a culture medium and centrifuged at 600 × g at room temperature for 5 minutes. After centrifugation, the supernatant was removed, the cells were suspended in 1 mL of the culture medium, and the number of cells was counted. Thereafter, the cells were diluted with a culture medium, seeded at 200 µL/well (4.7 × 10⁴ cells/cm²), and cultured under the conditions of 37°C and 5% CO₂. After culturing, images were acquired by imaging with IncuCyte S3 (Sartorius, Incucyte S3) every 6 hours.

### <Evaluation of neurite length>

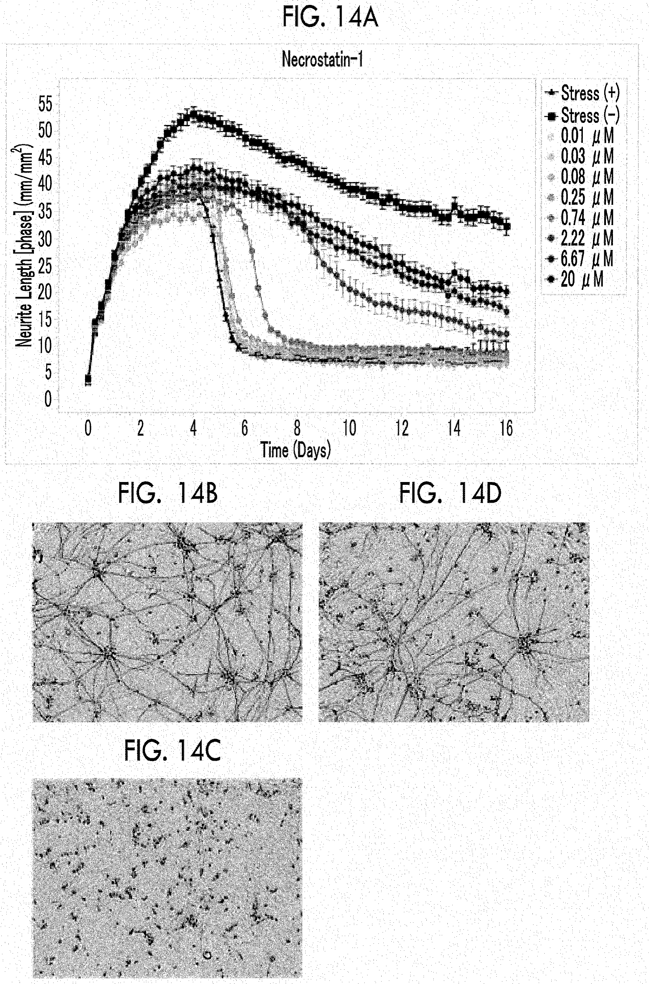

The neurite length was quantified using the Neurotrack software (Sartorius, 9600-0010) of IncuCyte S3. Fig. 14 shows the results of quantifying the neurite length and images of representative cells.

The nerve cells (Stress(+) group) (▲) cultured in the culture medium not containing an antioxidant were damaged after about 4 days of culture, and the sum of neurite lengths was reduced. On the other hand, in the group (•) treated with Necrostatin-1 known as a necroptosis inhibitor, the reduction in the sum of the neurite lengths was suppressed in a concentration-dependent manner. From this, it was shown that the nerve damage of the present evaluation system was caused by necroptosis.

### Test Example 7: Evaluation of compound (ferroptosis inhibitor) for oxidative stress-induced neuronal damage

### <Plate coating>

A solution obtained by diluting iMatrix-511 silk (Matrixome, 892021) 16.7 times with PBS was added to a 96-well plate (Corning, 356461) coated with PDL at 70 µL/well, and incubated at 37°C for 3 hours to 72 hours.

### <Preparation of assay plate>

As a culture medium containing an antioxidant, a culture medium (Stress(-)) in which DMSO was added to a +AO culture medium to a final concentration of 0.1% by volume (v/v) was used, as a culture medium not containing an antioxidant, a culture medium (Stress(+)) in which DMSO was added to a -AO culture medium to a final concentration of 0.1% by volume (v/v), and as the culture medium of the drug evaluation group, a culture medium in which a DMSO-dissolved compound was added to the -AO culture medium to a concentration of 0.1% by volume (v/v) (0.004 to 20 µM) was used.

**[Table 8]**

| +AO culture medium | |
|---|---|
| | mL |
| DMEM/F12 (Thermo Fisher Scientific, 11320-033) | 4.9 |
| B-27 Supplement (50X), serum free (Thermo Fisher Scientific, 17504-044) | 0.1 |

| -AO culture medium | |
|---|---|
| | mL |
| DMEM/F12 (Thermo Fisher Scientific, 11320-033) | 9.8 |
| B-27 Supplement (50X), minus antioxidants (Thermo Fisher Scientific. 10889-038) | 0.2 |

### <Cell seeding>

The nerve cells (considered to be cortical excitatory neurons) produced by forcibly expressing the Ngn2 genes from the iPS cells was thawed in a warm bath at 37°C. After melting, the cells were added to a culture medium and centrifuged at 600 × g at room temperature for 5 minutes. After centrifugation, the supernatant was removed, the cells were suspended in 1 mL of the culture medium, and the number of cells was counted. Thereafter, the cells were diluted with a culture medium, seeded at 200 µL/well (4.7 × 10⁴ cells/cm²), and cultured under the conditions of 37°C and 5% CO₂. After culturing, images were acquired by imaging with IncuCyte S3 (Sartorius, Incucyte S3) every 6 hours.

### <Evaluation of neurite length>

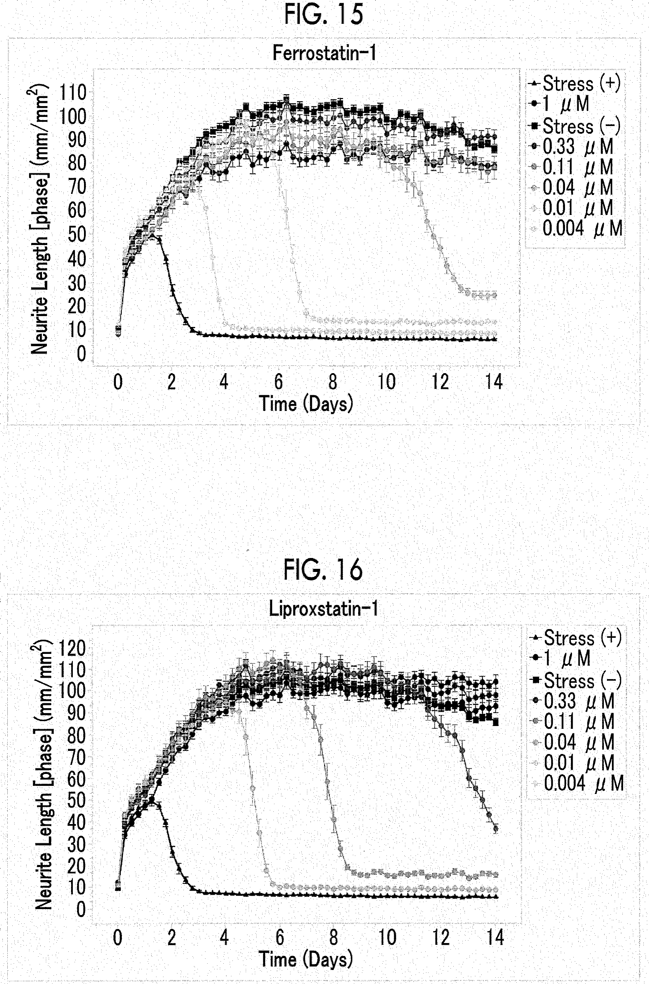

The neurite length was quantified using the Neurotrack software (Sartorius, 9600-0010) of IncuCyte S3. The results of quantifying the neurite length are shown in Figs. 15 to 17.

The nerve cells (Stress(+) group) (▲) cultured in the culture medium not containing an antioxidant were damaged after about 1 day of culture, and the sum of neurite lengths was reduced. On the other hand, in the group (•) treated with the ferroptosis inhibitor, the reduction in the sum of the neurite lengths was suppressed in a concentration-dependent manner for all the compounds. From these results, it was shown that the nerve damage of the present evaluation system was caused by ferroptosis.

### Test Example 8: Compound screening using oxidative stress-induced neuronal damage evaluation system

### <Plate coating>

A solution obtained by diluting iMatrix-511 silk (Matrixome, 892021) 16.7 times with PBS was added to a 96-well plate (Corning, 356461) coated with PDL at 70 µL/well, and incubated at 37°C for 3 hours to 72 hours.

### <Preparation of assay plate>

As the compound library, StemSelect Library compounds (Merck, 569774) were used. As a culture medium containing an antioxidant, a culture medium (Stress(-)) in which DMSO was added to a +AO culture medium to a final concentration of 0.1% by volume (v/v) was used, as a culture medium not containing an antioxidant, a culture medium (Stress(+)) in which DMSO was added to a -AO culture medium to a final concentration of 0.1% by volume (v/v), and as the culture medium of the drug evaluation group, a culture medium in which a DMSO-dissolved compound was added to the -AO culture medium to a concentration of 0.1% by volume (v/v) was used.

### [Table 9]

### <Cell seeding>

Frozen cells (iCell motor neurons, FCDI, C1048) were thawed in a warm bath at 37°C. After melting, the cells were added to a culture medium and centrifuged at 600 × g at room temperature for 5 minutes. After centrifugation, the supernatant was removed, the cells were suspended in 1 mL of the culture medium, and the number of cells was counted. Thereafter, the cells were diluted with a culture medium, seeded at 200 µL/well (4.7 × 10⁴ cells/cm²), and cultured under the conditions of 37°C and 5% CO₂. After culturing, images were acquired by imaging with IncuCyte S3 (Sartorius, Incucyte S3) every 6 hours.

### <Evaluation of neurite length>

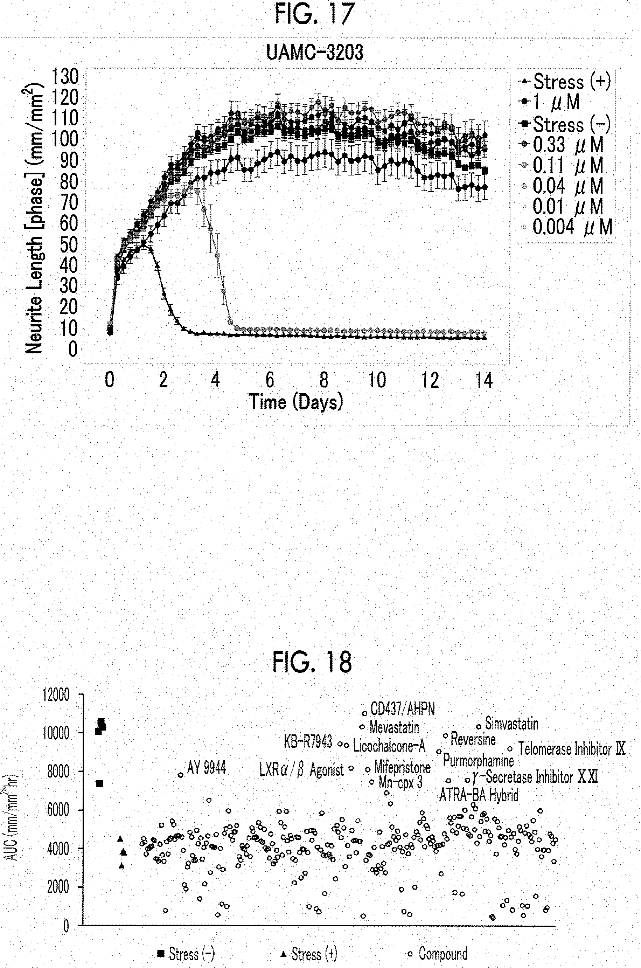

The neurite length was quantified using the Neurotrack software (Sartorius, 9600-0010) of IncuCyte S3. The area under the curve (AUC) of the curve of the neurite length over time for 14 days was calculated, and the effects of the drug were compared. That is, a large AUC indicates that the retraction of the neurite is suppressed (the nerve damage is suppressed). Fig. 18 shows each compound and the AUC values. The AUC of the nerve cells (Stress(+) group) (▲) cultured in the culture medium not containing an antioxidant was reduced as compared with the nerve cells (Stress(-) group) (▪) cultured in the culture medium containing an antioxidant. In the group (∘) in which the compound was treated, the reduction in AUC was strongly suppressed by CD437/AHPN, Simvastatin, Mevastatin, Reversine, KB-R7943, Licochalcone-A, Telomerase Inhibitor IX, Purmorphamine, LXRα/β Agonist, Mifepristone, AY 9944, γ-Secretase Inhibitor XXI, ATRA-BA Hybrid, and Mn-cpx 3.

### Test Example 9: Evaluation of ALS therapeutic drug (edaravone) for oxidative stress-induced neuronal damage

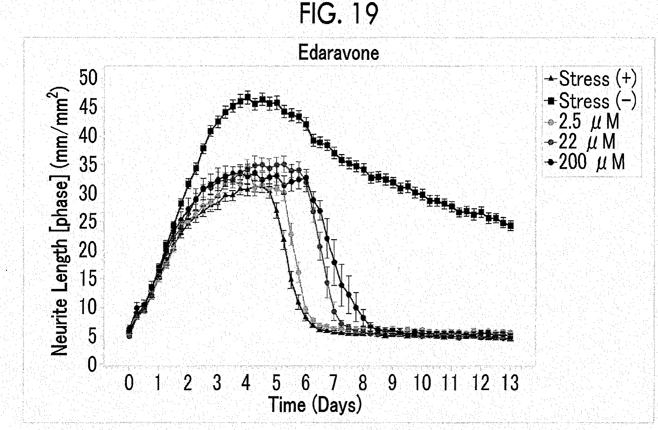

The effect of edaravone, which was used as a therapeutic drug for ALS, on oxidative stress-induced neuronal damage was evaluated by the same method as in Test Example 7. Fig. 19 shows the results of quantifying the neurite length. The nerve cells (Stress(+) group) (▲) cultured in the culture medium not containing an antioxidant were damaged after about 4 days of culture, and the sum of neurite lengths was reduced. In the group (•) treated with edaravone, the reduction in the sum of the neurite lengths was suppressed in a concentration-dependent manner. From this, it was shown that the drug candidates for treating ALS can be screened by the present evaluation system.

## Claims

1. A production method of nerve cells damaged by oxidative stress, the production method comprising:
a step a of seeding nerve cells obtained by differentiation from a human-derived pluripotent stem cell, at a cell density of 20.0 × 10⁴ cells/cm² or less by using a culture medium that substantially does not contain an antioxidant and substantially does not contain an oxidant; and
a step b of culturing the nerve cells by using the culture medium that substantially does not contain an antioxidant and substantially does not contain an oxidant.

2. The production method according to claim 1,
wherein the cell density is 0.2 × 10⁴ cells/cm² or more and 20.0 × 10⁴ cells/cm² or less.

3. The production method according to claim 1 or 2,
wherein the human-derived pluripotent stem cell is a pluripotent stem cell having no mutation in a disease-related gene.

4. The production method according to claim 1 or 2,
wherein the nerve cells are motor neurons, cortical excitatory neurons, or substantia nigra neurons.

5. The production method according to claim 1 or 2,
wherein the nerve cells damaged by oxidative stress satisfy at least one or more of the following (i) to (iv):
(i) a marker related to the oxidative stress is positive,
(ii) neurites are retracted,
(iii) necroptosis is induced, and
(iv) ferroptosis is induced.

6. A culturing method for cells, comprising:
a step a of seeding nerve cells obtained by differentiation from a human-derived pluripotent stem cell, at a cell density of 20.0 × 10⁴ cells/cm² or less by using a culture medium that substantially does not contain an antioxidant and substantially does not contain an oxidant; and
a step b of culturing the nerve cells by using the culture medium that substantially does not contain an antioxidant and substantially does not contain an oxidant.

7. The culturing method according to claim 6,
wherein the cell density is 0.2 × 10⁴ cells/cm² or more and 20.0 × 10⁴ cells/cm² or less.

8. The culturing method according to claim 6 or 7,
wherein the human-derived pluripotent stem cell is a pluripotent stem cell having no mutation in a disease-related gene.

9. The culturing method according to claim 6 or 7,
wherein the nerve cells are motor neurons, cortical excitatory neurons, or substantia nigra neurons.

10. Nerve cells obtained by the production method according to claim 1, which satisfy at least one of the following (i) to (iv):
(i) a marker related to the oxidative stress is positive,
(ii) neurite length is retracted,
(iii) necroptosis is induced, and
(iv) ferroptosis is induced.

11. The cells according to claim 10,
wherein the nerve cells are motor neurons, cortical excitatory neurons, or substantia nigra neurons.

12. An evaluation method for a test substance, comprising:
bringing the test substance into contact with the nerve cells according to claim 10.

13. The evaluation method for a test substance according to claim 12, further comprising:
producing the nerve cells according to claim 10 by the method according to claim 1,
wherein the test substance is brought into contact with the nerve cells.

14. A screening method for a drug for prevention and/or treatment of a neurodegenerative disease, the method comprising:
bringing a test substance into contact with the nerve cells according to claim 10.

15. The screening method according to claim 14, further comprising:
producing the nerve cells according to claim 10 by the method according to claim 1,
wherein the test substance is brought into contact with the nerve cells.

16. The screening method according to claim 14,
wherein the neurodegenerative disease is selected from the group consisting of Alzheimer's disease (AD), spinocerebellar degeneration, frontotemporal lobar degeneration (FTLD), Parkinson's disease, amyotrophic lateral sclerosis (ALS), dementia with Lewy bodies, Huntington's disease, and Niemann-Pick disease.

17. The screening method according to claim 14, further comprising, after bringing the test substance into contact with the nerve cells according to claim 10:
(A) a step of culturing the nerve cells that has been brought into contact with the test substance and nerve cells, used as a control, that have never been brought into contact with the test substance;
(B) a step of measuring nerve damage in the nerve cells; and
(C) a step of selecting, as a candidate for a drug for prevention and/or treatment of a neurodegenerative disease, a test substance that suppresses the nerve damage in comparison with the nerve cells, used as a control, that have never been brought into contact with the test substance.

18. The screening method according to claim 17,
wherein the step (B) is a step of measuring the number and/or the neurite length of the nerve cells obtained in the step (A).

19. The method according to claim 17 or 18,
wherein the step (C) is a step of selecting, as a candidate for a drug for prevention and/or treatment of a neurodegenerative disease, a test substance that has been brought into contact with the nerve cells and causes the number and/or the neurite length of the nerve cells to be greater than that in the control.

20. A screening method for a necroptosis inhibitor, comprising:
(1) a step of bringing a test substance into contact with the nerve cells according to claim 10;
(2) a step of culturing the nerve cells that has been brought into contact with the test substance in the step (1) and nerve cells, used as a control, that have never been brought into contact with the test substance;
(3) a step of measuring nerve damage in the nerve cells; and
(4) a step of selecting, as a candidate for a necroptosis inhibitor, a test substance that suppresses the nerve damage in comparison with the nerve cells, used as a control, that have never been brought into contact with the test substance.

21. The screening method according to claim 20, further comprising:
producing the nerve cells according to claim 10 by the method according to claim 1,
wherein the test substance is brought into contact with the nerve cells.

22. A screening method for a ferroptosis inhibitor, comprising:
(1) a step of bringing a test substance into contact with the nerve cells according to claim 10;
(2) a step of culturing the nerve cells that has been brought into contact with the test substance in the step (1) and nerve cells, used as a control, that have never been brought into contact with the test substance;
(3) a step of measuring nerve damage in the nerve cells; and
(4) a step of selecting, as a candidate for a ferroptosis inhibitor, a test substance that suppresses the nerve damage in comparison with the nerve cells, used as a control, that have never been brought into contact with the test substance.

23. The screening method according to claim 22, further comprising:
producing the nerve cells according to claim 10 by the method according to claim 1,
wherein the test substance is brought into contact with the nerve cells.
